# EUROPEAN PATENT APPLICATION

(11) **EP 1 938 814 A1**
(43) Date of publication of application: **02.07.2008**
(21) Application number: 06811858.7
(22) Date of filing: 17.10.2006
(51) Int. Cl.: A61K 31/20, A61K 31/428, A61K 45/00, A61P 11/00, A61P 21/00, A61P 25/02

(54) **PHARMACEUTICAL FOR PROTECTION OF MOTOR NERVE IN PATIENT WITH AMYOTROPHIC LATERAL SCLEROSIS**

(30) Priority: 18.10.2005 JP 2005302475
(71) Applicant: ONO PHARMACEUTICAL CO., LTD., Osaka-shi, Osaka 541-8526 (JP)
(72) Inventor: NISHII, Mikio, Osaka-shi Osaka 541-8564 (JP); FUNAKOSHI, Yosuke, Osaka-shi Osaka 541-8564 (JP); IMAGAWA, Masayuki, Osaka-shi Osaka 541-8564 (JP); ABE, Shinichiro, Osaka-shi Osaka 541-8564 (JP); KUWAYAMA, Tomohiro, Osaka-shi Osaka 541-8564 (JP)
(74) Representative: Henkel, Feiler & Hänzel
(86) International application number: PCT/JP2006/320603
(87) International publication number: WO 2007/046347

(57) **Abstract**

The present invention relates to an agent for protecting a motor nerve of a patient with amyotrophic lateral sclerosis, which comprises a combination of (a) (2R)-2-propyloctanoic acid or a salt thereof and (b) a therapeutic agent for amyotrophic lateral sclerosis such as riluzole. An agent comprising a combination of (a) (2R)-2-propyloctanoic acid which is orally administered once a day in an amount per dose of about 1200 mg and (b) riluzole which is orally administered twice a day in an amount per dose of about 50 mg is useful for protecting a motor nerve in a patient(s) with amyotrophic lateral sclerosis. The agent can also suppress the respiratory disability associated with the progression of the disease condition and improve the survival rate of the patient(s).

## Description

### TECHNICAL FIELD

The present invention relates to a useful method for protecting a motor nerve of a patient with amyotrophic lateral sclerosis, and an agent for use therein.

More specifically, the present invention relates to a method for administrering (2R)-2-propyloctanoic acid or a salt thereof in combination with therapeutic agent for amyotrophic lateral sclerosis such as riluzole, to the patient with amyotrophic lateral sclerosis, in order to protect motor nerve in patient with amyotrophic lateral sclerosis, suppress muscular weakness and obtain valuable effects such as suppression of respiratory disability and/or extension of survival period, and an agent for use therein.

### BACKGROUND ART

Amyotrophic lateral sclerosis (hereinafter sometimes abbreviated as ALS) is an intractable and progressive neurodegenerative disease which is caused by the selective degeneration of upper and lower motor neurons. Due to weakness of skeletal muscles in the four limbs and trunk, a patient with the onset of ALS needs bed rest. Also, the patient suffers from dysphagia and respiratory failure due to muscular weakness of swallowing muscles and respiratory muscles. Finally, the patient dies from respiratory muscle paralysis, *etc*. Although the morbidity of ALS is as low as about from 2 to 7 people per 100,000 population, many ALS patients die within 2 to 3 years and most of them die within 5 years. Accordingly, it has been urgently required to develop a method of treating the disease and a method of controlling the progression thereof.

It is suggested that the selective degeneration of motor neurons is caused by axonal transport injury, oxidative stress caused by free radicals, glutamic acid-induced neurotoxicity and so on (Cleaveland D.W., Neuron, 24, 515-520 (1999); Hatano Shinji, Jikken Igaku, 19, 2283-2288 (2001); Niwa Junichi, Saishin Igaku, 56, 1622-1631 (2001); and Rothstein J.D., Current Opinion in Neurobiology, 6, 679-687 (1996)). However, the onset mechanism thereof has never been clarified hitherto. Concerning therapeutic agents therefor, studies have been made on the therapeutic effects of an agent and a neurotrophic factor having a nerve protective activity, an agent having an inhibitory activity on glutamic acid, an agent having an inhibitory activity on caspase, an agent having a copper chelating activity and so on. However, nothing but riluzole (trade name: Rilutek), which is a glutamic acid receptor antagonist, has been approved and sold at present as a drug for ALS patients.

On the other hand, it is reported that (2R)-2-propyloctanoic acid is a compound which can improve the function of abnormally activated astrocytes and can be used as an agent for prevention or treatment of various cranial nerve diseases including cerebral stroke since it has an effect of reducing intracellular S100B (also known as S100β) content (see, for example, Journal of cerebral blood flow & metabolism, 22, 723-734, 2002: Non-Patent Document 1).

It is also known that pentanoic acid derivatives including (2R)-2-propyloctanoic acid have an effect of improving the function of astrocytes and are useful as an agent for improving brain function. For example, they can be used in treatment of Alzheimer's disease, ALS and so forth. Concerning dosing, it is reported that such a compound is orally administered to an adult in an amount per dose of from 1 to 1000 mg once to several times per day, or parenterally administered in an amount per dose of from 0.1 to 100 mg once to several times per day (see, for example, the specification of European Patent 0632008: Patent Document 1).

Also, it is known that (2R)-2-propyloctanoic acid or a salt thereof has an activity of promoting nerve regeneration and can be used in combination with a therapeutic agent for ALS such as riluzole. It is reported that (2R)-2-propyloctanoic acid or a salt thereof is orally administered to an adult in an amount per dose of from 1 µg to 5000 mg one to several times per day, or parenterally administered in an amount per dose of from 0.1ng to 500 mg one to several times per day (see, for example, WO 2005/032535 (Patent Document 2)).

However, in these documents, a dosing method, dose, dosing time and so on whereby the efficacy of (2R)-2-propyloctanoic acid or a salt thereof can be established on human ALS patients in practice are not described specifically at all. Particularly, it is never described therein that highly useful effects such as suppression of respiratory disability or improvement in survival rate can be achieved by administering (2R)-2-propyloctanoic acid in combination with riluzole to an ALS patient within a relatively short period of time from the onset of muscular weakness.
[Patent Document 1] Specification of European Patent 0632008
[Patent Document 2] International Publication Pamphlet WO2005/032535
[Non-Patent Document 1] Journal of cerebral blood flow & metabolism, 22, 723-734, 2002

### DISCLOSURE OF THE INVENTION

### PROBLEMS TO BE SOLVED BY THE INVENTION

Although ALS is an intractable, progressive and fatal disease which cannot be completely cured when once it onsets, riluzole is the sole agent which has been approved as a therapeutic agent therefor. However, the data of clinical trials conducted in Japan during 1993 to 1996 clearly indicate that no sufficient and desirable efficacy can be obtained even when riluzole is used. According to studies of the inventors of the present invention, (2R)-2-propyloctanoic acid cannot exert any satisfactory effect on ALS patients when it is used alone.

Under these circumstances, it has been required to develop an agent, which is safe and sufficiently efficacious from a clinical viewpoint, for suppression of the respiratory disability of ALS patients or improving the survival rate to relieve the pain of ALS patients who would die within only several years after the onset.

### MEANS FOR SOLVING THE PROBLEMS

The inventors of the present invention have conducted intensive studies to solve the above-described problems. As a result, they found out that clinically sufficient efficacy, *e.g.*, suppression of respiratory disability and improvement in survival rate, can be highly safely obtained by administering 1200 mg per day of (2R)-2-propyloctanoic acid to ALS patients (in particular, ALS patients within about 14 months after the onset of muscular weakness) under the standard dosing of riluzole. Based on the finding, the inventors of the present invention have further conducted detailed studies and consequently accomplished the present invention.

Accordingly, the present invention relates to:
[1] An agent for protecting a motor nerve of a patient with amyotrophic lateral sclerosis, which comprises a combination of (a) (2R)-2-propyloctanoic acid or a salt thereof and (b) a therapeutic agent for amyotrophic lateral sclerosis;
[2] The agent according to above [1], which is used for suppressing muscular weakness;
[3] The agent according to above [2], which is used for suppressing respiratory disability;
[4] The agent according to above [2], which is used for extending survival period;
[5] The agent according to above [1], which is for oral administration;
[6] The agent according to above [1], wherein the therapeutic agent for amyotrophic lateral sclerosis is riluzole;
[7] The agent according to above [6], wherein the amount per dose of riluzole is 50 mg;
[8] The agent according to above [1], wherein the amount per dose of (2R)-2-propyloctanoic acid or a salt thereof is from 300 mg to 2400 mg in terms of (2R)-2-propyloctanoic acid;
[9] The agent according to above [8], wherein the amount per dose of (2R)-2-propyloctanoic acid or a salt thereof is 300 mg, 600 mg, 900 mg, 1200 mg, 1500 mg, 1800 mg, 2100 mg or 2400 mg in terms of (2R)-2-propyloctanoic acid;
[10] The agent according to above [9], wherein the amount per dose of (2R)-2-propyloctanoic acid or a salt thereof is 1200 mg in terms of (2R)-2-propyloctanoic acid;
[11] The agent according to above [6], wherein the amount per daily dose of (2R)-2-propyloctanoic acid or a salt thereof is from 1200 mg to 2400 mg in terms of (2R)-2-propyloctanoic acid and the amount per daily dose of riluzole is from 50 mg to 200 mg;
[12] The agent according to above [11], wherein the dosing time per day of (2R)-2-propyloctanoic acid or a salt thereof is from once to twice and the dosing time per day of riluzole is from one to four times;
[13] An agent for protecting a motor nerve of a patient with amyotrophic lateral sclerosis, which comprises a combination of (a) (2R)-2-propyloctanoic acid or a salt thereof and (b) riluzole and is administered for from one to five years;
[14] The agent according to above [13], which is administered for 12 months to 18 months;
[15] The agent according to above [1] or [13], wherein the patient with amyotrophic lateral sclerosis is a patient within 14 months after the onset of muscular weakness;
[16] An agent for suppressing respiratory disability and/or extending survival period of a patient with amyotrophic lateral sclerosis within 14 months after the onset of muscular weakness, which comprises a combination of (a) (2R)-2-propyloctanoic acid which is orally administered once a day in an amount per dose of 1200 mg and (b) riluzole which is orally administered twice a day in an amount per dose of 50 mg during the dosing period of from 12 months to 18 months;
[17] An agent which comprises a combination of (a) (2R)-2-propyloctanoic acid which is orally administered once a day in an amount per dose of 1200 mg and (b) riluzole which is orally administered twice a day in an amount per dose of 50 mg during the dosing period of from 12 months to 18 months to suppress respiratory disability and/or extend survival period of a patient with amyotrophic lateral sclerosis within 14 months after the onset of muscular weakness;
[18] A method for protecting a motor nerve of a patient with amyotrophic lateral sclerosis, which comprises orally administering a combination of (a) an effective amount of (2R)-2-propyloctanoic acid or a salt thereof and (b) an effective amount of a therapeutic agent for amyotrophic lateral sclerosis to the patient with amyotrophic lateral sclerosis;
[19] Use of a combination of (a) (2R)-2-propyloctanoic acid or a salt thereof and (b) a therapeutic agent for amyotrophic lateral sclerosis for producing an agent for protecting a motor nerve which is orally administered to a patient with amyotrophic lateral sclerosis;
and so forth.

### EFFECT OF THE INVENTION

According to the method of the present invention, it is possible to obtain effects of protecting the motor nerve of an ALS patient, particularly, an effect of suppressing muscular weakness including suppression of respiratory disability and improvement in survival rate. Moreover, other ALS symptoms can be ameliorated thereby. As a result, it becomes possible to exert effects of controlling the progression of the symptoms or improving the same in the ALS function evaluation scale as is shown in Examples hereinafter. By using the method according to the present invention, these symptoms can be improved. Additionally, the time until the attachment of a respirator can be prolonged, or the survival period can be extended. It is also possible to improve QOL.

### BEST MODE FOR CARRYING OUT THE INVENTION

In the present invention, (2R)-2-propyloctanoic acid is a compound represented by the formula (I): 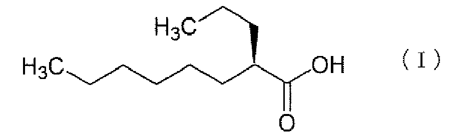 wherein represents β-configuration.

In the present invention, a preferable salt of (2R)-2-propyloctanoic acid is a pharmaceutically acceptable salt. Preferable pharmaceutically acceptable salt is a less-toxic water-soluble salt. Examples of a suitable salt of (2R)-2-propyloctanoic acid include salts with inorganic bases, salts with organic bases, salts with basic natural amino acids and so forth. Preferable examples of the salts with inorganic bases include an alkali metal salt (*e.g.*, a sodium salt, a potassium salt, a lithium salt, *etc.*), an ammonium salt (*e.g.,* a tetramethylammonium salt, a tetrabutylammonium salt, *etc.*) and the like. Preferable examples of the salts with organic bases include a salt with an alkylamine (*e.g.*, methylamine, dimethylamine, trimethylamine, triethylamine, *etc.*), a heterocyclic amine (*e.g.*, pyridine, picoline, piperidine, *etc.*), an alkanolamine (*e.g.*, ethanolamine, diethanolamine, triethanoalmine, *etc.*), dicyclohexylamine, N,N'-dibenzylethylenediamine, cyclopentylamine, benzylamine, dibenzylamine, phenethylamine, tris(hydroxymethyl)methylamine, N-methyl-D-glucamine and the like. The salt with a basic natural amino acid is not particularly limited, so long as it is a salt with a basic amino acid which occurs in nature and can be purified. It is preferable to use, for example, a salt with arginine, lysine, ornithine, histidine or the like.

(2R)-2-propyloctanoic acid or a salt thereof can be prepared by methods known *per se,* for example, the methods described in the specification of EP patent No.0632008, WO 99158513, WO 00/48982, the specification of JP patent No.3032447, the specification of JP patent No.3084345, WO 20031051852, WO 20031097851, WO 2004/092113, WO 2004/110972, WO 2005/105722, methods similar thereto, or the method described in Comprehensive Organic Transformations: A Guide to Functional Group Preparations, 2nd Edition (Richard C. Larock, John Wiley & Sons Inc., 1999) or by appropriate combinations of such methods. The reaction product may be purified by conventional purification methods, for example, by distillation under atmospheric or reduced pressure, high performance liquid chromatography, thin layer chromatography or column chromatography using silica gel or magnesium silicate, or by methods such as washing and recrystallization. Also, if necessary, the product may be subjected to additional treatments such as freeze-drying.

(2R)-2-propyloctanoic acid or a salt thereof to be used in the present invention is not limited to those which are substantially pure single substances, and they may contain impurities (*e.g.*, byproducts, solvents, starting materials, *etc.* originating form the preparation process, or decomposition products, *etc.*) within the scope acceptable as a medicinal bulk. The content of impurities acceptable as the medicinal bulk varies depending on whether the (2R)-2-propyloctanoic acid is used or a salt thereof is used, and also varies depending on the contained impurities. In the case of (2R)-2-propyloctanoic acid, for example, it is desirable that heavy metals (*e.g.*, lead, bismuth, cuprum, cadmium, antimony, tin, mercury, *etc.*) are about 20 ppm or less, the S-form as its optical isomer is about 1.49 % by mass or less, 2-propanol and heptane as the residual solvents are about 5000 ppm or less in total, and the moisture is about 0.2 % by mass or less. Particularly, (2R)-2-propyloctanoic acid having an optical purity of about 99 % e.e. or more, more particularly, (2R)-2-propyloctanoic acid having an optical purity of about 99.3 % e.e. or more, is suitable as the (2R)-2-propyloctanoic acid to be used in the present invention.

An object of the present invention is to protect the motor nerve of an ALS patient. More particularly, an object of the present invention is to protect the motor nerve of an ALS patient; suppress muscular weakness; suppress respiratory disability and/or extend survival period. In the present invention, a method which comprises administering to an ALS patient (2R)-2-propyloctanoic acid or a salt thereof as described above (preferably (2R)-2-propyloctanoic acid) in an effective amount, preferably from about 50 mg to about 300 mg (*e.g.*, about 50 mg, about 100 mg, about 150 mg, about 300 mg, about 600 mg, about 900 mg, about 1200 mg, about 1500 mg, about 1800 mg, about 2100 mg, about 2400 mg, about 2700 mg or about 3000 mg) per dose in terms of (2R)-2-propyloctanoic acid, more preferably from about 300 mg to about 2400 mg (*e.g.*, about 300 mg, about 600 mg, about 900 mg, about 1200 mg, about 1500 mg, about 1800 mg, about 2100 mg or about 2400 mg) per dose in terms of (2R)-2-propyloctanoic acid, and particularly preferably about 1200 mg per dose in terms of (2R)-2-propyloctanoic acid in combination with a therapeutic agent for ALS represented by riluzole (hereinafter sometimes abbreviated as the method of the present invention) and an agent for use therein are disclosed.

In the present invention, (2R)-2-propyloctanoic acid or a salt thereof is used in combination with a therapeutic agent for ALS represented by riluzole. The combination method may be an arbitrary one. For example, (a) (2R)-2-propyloctanoic acid or a salt thereof and (b) a therapeutic agent for ALS may be contained in a single pharmaceutical composition (i.e., a so-called combination preparation). Alternatively, (a) (2R)-2-propyloctanoic acid or a salt thereof and (b) a therapeutic agent for ALS may be contained in separate pharmaceutical compositions. In the case where (a) (2R)-2-propyloctanoic acid or a salt thereof and (b) a therapeutic agent for ALS are contained in separate pharmaceutical compositions, these compositions may be administered either simultaneously or with some time-lag. In the case where these compositions are administered with some time-lag, (a) (2R)-2-propyloctanoic acid or a salt thereof may be administered in advance and followed by administration of (b) a therapeutic agent for ALS. Alternatively, (b) a therapeutic agent for ALS may be administered in advance and followed by (a) (2R)-2-propylactanoic acid or a salt thereof. The dosing route and dosing time per day may be either the same or different. Moreover, the weight ratio of (a) (2R)-2-propyloctanoic acid or a salt thereof to (b) a therapeutic agent for ALS is not particularly limited. In the present invention, it is sufficient that (a) (2R)-2-propyloctanoic acid or a salt thereof is used in combination with a therapeutic agent for ALS. If desired, it may be further combined with other components of agent (for example, an amino acid (*e.g.*, leucine, isoleucine, valine, threonine, *etc.*), dextromethorphan, gabapentin, lamotrigine, N-acetylcysteine, vitamin C, vitamin B (*e.g.*, vitamin B₁, B₂, B₆, B₁₂, *etc.*), vitamin E, IGF-I (insulin-like growth factor-1), CNTF (ciliary neurotrophic factor), GH (growth hormone), TRH (thyrotropin releasing hormone), BDNF (brain-derived neurotrophic factor), ganglioside, cyclosporine, selegiline, physostigmine, diaminopyridine, modified snake venom, interferon β, atropine, trihexyphenidyl, scopolamine, botulinum toxin A, amitriptyline, fluvoxamine, opioids (*e.g.*, morphine, buprenorphine hydrochloride, *etc.*), chlorpromazine, diazepam, midazolam, *etc.*).

As the therapeutic agent for ALS in the present invention, any drug which has been marketed or developed targeting ALS or has an ALS-targeting mechanism can be used without particular limitation. Examples of such agents include riluzole, edaravone, arimoclomol, xaliproden hydrochloride, TCH-346, superoxide dismutase, pentoxifylline, T-588, glatiramer acetate, PYM-50018, EHT-0202, EP-0011, *etc.* It is preferable to use, for example, riluzole, edaravone, arimoclomol, xaliproden hydrochloride, TCH-346, superoxide dismutase, pentoxifylline, T-588, glatiramer acetate, *etc.* It is more preferable to use, for example, riluzole, edaravone, xaliproden hydrochloride, *etc.* It is particularly preferable to use, for example, riluzole, edaravone, *etc.* Among all, riluzole is most appropriately usable as the therapeutic agent for ALS in the present invention, since it has been approved as an agent targeting ALS as discussed above and used in a number of countries in the world.

In the method of the present invention, (2R)-2-propyloctanoic acid or a salt thereof is used in combination with a therapeutic agent for ALS represented by, for example, riluzole, i.e., as "an agent for protecting a motor nerve of a patient with ALS which comprises a combination of (a) (2R)-2-propyloctanoic acid or a salt thereof and (b) a therapeutic agent for ALS" (hereinafter sometimes abbreviated as the agent of the present invention).

In the present invention, the dosing route may be an arbitrary one as long as an effective amount of the agent can be administered into the living body. For example, systemic administration such as oral administration or intravenous administration is preferable. As described above, the agent of the present invention may comprise (a) (2R)-2-propyloctanoic acid or a salt thereof and (b) a therapeutic agent for ALS, which are contained in a single pharmaceutical composition (i.e., a so-called combination preparation). Alternatively, (a) (2R)-2-propyloctanoic acid or a salt thereof and (b) a therapeutic agent for ALS may be contained in separate pharmaceutical compositions. In the case where (a) (2R)-2-propyloctanoic acid or a salt thereof and (b) a therapeutic agent for ALS are contained in a single pharmaceutical composition, the pharmaceutical composition may be systemically administered by oral administration or intravenous administration. In the case where (a) (2R)-2-propyloctanoic acid or a salt thereof and (b) a therapeutic agent for ALS are contained in separate pharmaceutical compositions, the agent of the present invention can be systemically administered by any one of the following four combinations, i.e., (1) the combination of the oral administration of a pharmaceutical composition containing (a) (2R)-2-propyloctanoic acid or a salt thereof and the oral administration of (b) a therapeutic agent for ALS; (2) the combination of the oral administration of a pharmaceutical composition containing (a) (2R)-2-propyloctanoic acid or a salt thereof and the intravenous administration of (b) a therapeutic agent for ALS; (3) the combination of the intravenous administration of a pharmaceutical composition containing (a) (2R)-2-propyloctanoic acid or a salt thereof and the oral administration of (b) a therapeutic agent for ALS; and (4) the combination of the intravenous administration of a pharmaceutical composition comprising (a) (2R)-2-propyloctanoic acid or a salt thereof with the intravenous administration of (b) a therapeutic agent for ALS. Among these dosing methods, it is preferable in the present invention to orally administer both of a pharmaceutical composition comprising (a) (2R)-2-propyloctanoic acid or a salt thereof and a pharmaceutical composition comprising (b) a therapeutic agent for ALS (the agent of the present invention used for oral administration).

With regard to dosing amount and dosing timing in the case of using riluzole as a therapeutic agent for ALS in the agent of the present invention, for example, it is preferable to administer riluzole in an amount per dose of from about 10 mg to about 200 mg 1 to 4 times per day. It is more preferable to administer riluzole in an amount per dose of about 50 mg 1 to 4 times per day (i.e., range of the daily dose of riluzole is from about 50 mg to about 200 mg). Specifically, the dosage regimen of riluzole for monotherapyof ALS, namely, to take riluzole in an amount per dose of 50 mg twice per day, *i.e.,* to take riluzole before breakfast and before dinner in a daily dose of 100 mg is particularly appropriate.

In the agent of the present invention, the dose of (2R)-2-propyloctanoic acid or a salt thereof may be an arbitrary amount as long as (2R)-2-propyloctanoic acid or a salt thereof can exert its efficacy without showing any remarkable toxicity *in vivo.* It is preferable that the amount per dose in terms of (2R)-2-propyloctanoic acid is from about 50 mg to about 3000 mg, more preferably from about 300 mg to about 2400 mg per dose, particularly preferably about 300 mg, about 600 mg, about 900 mg, about 1200 mg, about 1500 mg, about 1800 mg, about 2100 mg or about 2400 mg per dose, and still preferably about 1200 mg per dose.

In the agent of the present invention, the dosing timing of (2R)-2-propyloctanoic acid or a salt thereof may be appropriately selected in view of the amount per dose of (2R)-2-propyloctanoic acid or a salt thereof as described above and pharmacokinetics in the living body to which it has been administered (*e.g.*, about 1 to about 4 times per day). In the case of orally administering about 1200 mg per dose of (2R)-2-propyloctanoic acid, for example, the dosing timing is preferable once or twice a day. Namely, it is preferable to administer about from 1200 mg to about 2400 mg per day of (2R)-2-propyloctanoic acid and once a day is still preferable. It is particularly preferable to administer the drug once a day in the morning.

As discussed above, the method of the present invention and the agent of the present invention can be used in order to, for example, protect a motor nerve of an ALS patient. Specifically, they can be used for an ALS patient to suppress muscular weakness; suppress respiratory disability; extend survival period, *etc.*
The term "muscular weakness" as used herein means weakening in muscle strength in the skeletal muscles which is the main symptom of ALS. The term "respiratory disability" as used herein means lowering in respiratory function (including respiratory paralysis) caused by weakening in muscle strength in the skeletal muscles which participate in respiration (respiratory muscles). The term "motor nerve protection" means to protect motor nerve from motor nerve disorders caused by the disease ALS. The method of the present invention and the agent of the present invention can protect motor nerve to suppress weakening in muscle strength in the skeletal muscles.

In the method of the present invention, the dosing period of the agent of the present invention may be an arbitrary period of time as long as the motor nerve protective effects (*e.g.*, clinical therapeutic effects or an effect recognizable as an effect of controlling progression of symptoms such as an effect of suppressing muscular weakness, an effect of suppressing respiratory disability or an effect of extending survival period) on an ALS patient can be observed within the period.
In the present invention, the term "therapy" means a treatment intended to heal the pathologic conditions, while the expression "to control progression of symptoms" means to control progression/worsening of symptoms and inhibit the progress of the disease. Specifically, the dosing period may be a short period such as several months (*e.g.*, from about 2 or 3 months to about 6 months) or within one year (*e.g.*, about 1 to about 12 months). However, to achieve preferable motor nerve protection effects, it is preferable to administer the agent over longer than one year. The dosing period is more preferably from about 1 year to about 7 years (from about 12 months to about 84 months), particularly preferably from about 1 year to about 5 years (from about 12 months to about 60 months), especially preferably, from about 1 year to about 3 years (about 12 months to about 36 months). By administering the agent for at least about one year to about one and half year (about 12 months to about 18 months), more preferably for about one year (about 12 months), it is possible to achieve highly useful motor nerve protective effects such as suppression of respiratory disability and improvement in survival rate, as will be indicated in Examples hereinafter. It should be understood that the dosing period as described herein means the time wherein a combination of (a) (2R)-2-propyloctanoic acid or a salt thereof and (b) a therapeutic agent for ALS is used. Namely, before or after the dosing period as described above, there may be period for single administration of some agent.

It can be determined by a publicly known method whether or not the motor nerve protective effect can be exerted on an ALS patient by the administration of the agent of the present invention. For example, the determination is possible by evaluating respiratory function, functional status, muscle strength, index of serious respiratory failure, survival rate and so on as will be shown in Examples hereinafter. Additionally, any method can be used as long as the severity of ALS symptoms can be determined, Namely, the determination is possble depending on, for example, ability of independent ambulation, occurrence of upper limb dysfunction, practice of tracheotomy, respirator attachment, time until intubation or tracheotomy for respirator attachment (the starting date can be arbitrary selected from among, for example, the starting date of the administration of the agent of the present invention, the date of the first awareness of ALS symptoms such as muscular weakness and so on), practice of tubal feeding, manual muscle test (MMT), modified Japanese Limb Norris scale (21 items), Norris bulbar scale (13 items), grasping power, back muscle strength, pinching power, forced vital capacity (FVC), forced expiratory volume in the first second (FEV1), physiotherapeutic test, QOL (*e.g.,* QOL based on EQ-5D evaluation, *etc.*), time till death (survival period) (the starting date can be arbitrary selected from among, for example, the starting date of the administration of the agent of the present invention, the date of the first awareness of ALS symptoms such as muscular weakness and so on) or the like. Additionally, it is possible to measure S100B in the cerebrospinal fluid or blood serum and use it as an indication of the motor nerve protective effects obtained by the method of the present invention. S100B can be measured in accordance with a publicly known method. The motor nerve protective effects may be determined by using these methods either after the completion of the dosing period of the agent of the present invention or during the dosing period. When some efficacy is observed based on the standards for the determination in each case, anyway, more preferable effects can be obtained by starting again or continuing the administration of the agent of the present invention.

The effect of suppressing respiratory disability which is obtained by the administration of the agent of the present invention means the effect of slowing respiratory disability accompanying the progression of ALS, preventing the dysfunction or improving respiratory function. Whether the respiratory disability occurs or not may be determined based on the patient's subjective symptom. Alternatively, it may be determined depending on publicly known respiratory function evaluation items (*e.g.*, vital capacity, vital capacity in the first second, *etc.*)*.* It is preferable to make the determination based on vital capacity, as will be described in Examples hereinafter. In the present invention, vital capacity is sometimes expressed in "% to a predicted value" which is a value (percentage) determined by referring the "predicted value" calculated based on the body weight of a patient as to 100 %.

When a therapeutic agent for ALS represented by riluzole is administered, without using (2R)-2-propyloctanoic acid or a salt thereof, to an ALS patient (a patient within 14 months after the onset of muscular weakness as will be described hereinafter, in particular, a patient within 14 months after the onset of muscle showing a vital capacity of about 70 % or more of the predicted value at the starting of the administration of the agent of the present invention), the vital capacity of 12 months after the start of the administration is lowered to 50 % or less of the predicted value in many cases. However, by administering the agent of the present invention, the vital capacity of 12 months after the start of the administration can be maintained to about 50 % or more; preferably can be maintained about 55 % or more, more preferably can be maintained about 60 % or more; and particularly preferably can be maintained about 65 % or more of the predicted value.

Concerning the lowering level of vital capacity, when a therapeutic agent for ALS represented by riluzole is administered without using (2R)-2-propyloctanoic acid or a salt thereof, the lowering in the vital capacity of 12 months after the start of the administration is 50 % or more of the predicted value in many cases. However, by administering the agent of the present invention, the lowering in the vital capacity can be maintained within about 50 %, preferably within can be maintained about 45 %, more preferably can be maintained within about 40 % and particularly preferably can be maintained within about 35 %. The expression "the lowering in the vital capacity can be maintained within 35 % of the predicted value" as used herein means that, when the vital capacity before the administration is 90 % of the predicted value, the vital capacity of 12 months after the initiation of the administration is 55 % (=90-35) of the predicted value.

The effect of extending survival period which is obtained by administering the agent of the present invention means the effect of retarding death accompanying the progression of ALS. As will be shown in Examples hereinafter, the effect of extending survival period can be calculated as mortality or survival rate from the number of death cases within a definite period of time. By using the agent of the present invention, it is possible to obtain the effect of extending survival period in about 40 % to about 60 % of patients, in comparison with the case where it is not used (*i.e.,* administering a therapeutic agent for ALS represented by riluzole without administering (2R)-2-propyloctanoic acid or a salt thereof).

The method of the present invention is applied to an ALS patient. In the method according to the present invention, particulary, the method with the use of an agent comprising a combination of (a) (2R)-2-propyloctanoic acid which is administered once a day in an amount per dose of 1200 mg with (b) riluzole which is administered twice a day in an amount per dose of 50 mg is preferably applied to an ALS patient within a short period of time after the onset of muscular weakness (i.e., patient with ALS in the early stage), for example, a patient within about 12 months to about 15 months after the onset of muscular weakness, particulary, a patient within 14 months after the onset of muscular weakness, from among ALS patients. Among ALS patients within 14 months after the onset of muscular weakness, a patient within 14 months after the onset of muscular weakness who fulfills the inclusion criteria as will be shown in Examples hereinafter but does not fulfill the exclusion criteria is particularly preferable. The expression "the onset of muscular weakness" as used herein means that muscular weakness occurs as the subjective symptom of a patient or the timing thereof.

In the method of the present invention, it is preferred to combine preferable dosing periods, in addition to the patients. Namely, the method with the use of "an agent for suppressing respiratory disability and/or extending survival period of a patient with amyotrophic lateral sclerosis within about 14 months after the onset of muscular weakness which comprises a combination of (a) (2R)-2-propyloctanoic acid which is administered once a day in an amount per dose of about 1200 mg and (b) riluzole which is administered twice a day in an amount per dose of about 50 mg during the dosing period of from about 12 months to about 18 months" or "an agent which comprises a combination of (a) (2R)-2-propyloctanoic acid which is administered once a day in an amount per dose of about 1200 mg with (b) riluzole which is administered twice a day in an amount per dose of about 50 mg during the dosing period of from about 12 months to about 18 months to suppress respiratory disability and/or extend survival period of a patient with amyotrophic lateral sclerosis within about 14 months after the onset of muscular weakness" is preferable.

### [Toxicity]

As shown in Examples hereinafter, the toxicity of (2R)-2-propyloctanoic acid or a salt thereof is extremely low, so it can be considered to be sufficiently safe for medicinal uses.

### [Application to Medicament]

The agent for protecting a motor nerve of an ALS patient of the present invention which comprises a combination of (a) (2R)-2-propyloctanoic acid or a salt thereof and (b) a therapeutic agent for ALS can be used for ALS patients. By orally administering the agent in the dosing method and dose as specified in the present invention to an ALS patient, preferably to a patient with ALS in the early stage and particularly preferably to an ALS patient within 14 months after the onset of muscular weakness, preferable motor nerve protective effects can be exerted on the ALS patient. The Dosage and Administration of the present invention improves various symptoms of an ALS patient, particulary, a patient within a short period of time after the onset of muscular weakness. Therefore, they are suitable for obtaining, for example, an effect of improving respiratory function, an effect of improving survival rate and so on.

When the agent of the present invention is administered to a patient, an agent wherein (a) (2R)-2-propyloctanoic acid or a salt thereof and (b) a therapeutic agent for ALS are contained in separate pharmaceutical compositions or an agent wherein (a) (2R)-2-propyloctanoic acid or a salt thereof and (b) a therapeutic agent for ALS are contained in a single pharmaceutical composition is used. As described above, any composition can be used in the method of the present invention as long as the effective amounts of the agent can be administered to the living body. However, it is preferable to use an agent wherein (a) (2R)-2-propyloctanoic acid or a salt thereof and (b) a therapeutic agent for ALS are contained in separate pharmaceutical compositions. Particularly, in the case of using riluzole as the therapeutic agent for ALS, it is preferable to use a publicly known tablet (for example, a tablet containing 50 mg of riluzole (*e.g.*, Rilutek (trade name), *etc.*)*, etc.*) as a pharmaceutical composition comprising the therapeutic agent for ALS.

As the pharmaceutical composition comprising (2R)-2-propyloctanoic acid or a salt thereof, for example, solid compositions or liquid compositions for oral administration can be preferably used.

Solid compositions for oral administration include compressed tablets, pills, capsules, powders and granules. Capsules include hard capsules and soft capsules.

In such a solid composition for oral administration, the agent is used either as such or mixed with an excipient (*e.g.*, lactose, mannitol, glucose, microcrystalline cellulose, starch, *etc.*), a binder (*e.g.*, hydroxypropylcellulose, polyvinylpyrrolidone, magnesium metasilicate aluminate, *etc.*), a disintegrating agent (*e.g.*, cellulose calcium glycolate, *etc.*), a lubricant (*e.g.*, magnesium stearate, *etc.*) a stabilizer, a dissolution aid (*e.g.*, glutamic acid, aspartic acid, *etc.*) and the like and then formulated into a preparation in accordance with a conventional method. If necessary, it may be coated with a coating agent (*e.g.*, sucrose, gelatin, hydroxypropylcellulose, hydroxypropylmethylcellulose phthalate, *etc.*). It is also possible to coat it with two or more layers. A capsule made of an absorbable substance such as gelatin also falls within the scope.

A soft capsule preparation for oral administration (hereinafter sometimes abbreviated as "a capsule preparation used in the present invention") can be produced in accordance with a publicly known method of producing soft capsule preparations. Specifically, the soft capsule preparation can be obtained by subjecting the agent and a coating solution to a publicly known encapsulation method such as the stamping method (*e.g.,* the rotary die method, *etc.*) or the dropping method (*e.g.*, the seamless capsule method, *etc.*) followed by drying the obtained capsules. The capsule preparation used in the present invention may be either a seamless soft capsule preparation or a seamed soft capsule preparation. Methods of producing seamless capsule preparations are described in detail in publicly known documents, for example, Baiosaiensu to Indasutori (Bioscience & Industry), 58(7), 31 to 34 (2000). Since the basic method of producing a seamless capsule preparation has been industrially used, mass production can be easily carried out.

Similar to commonly used soft capsule preparations, the capsule preparation used in the present invention is composed of an inner solution and a capsule shell. The capsule shell may comprise a substance which is called a capsule base and serves as the main component of the capsule shell (for example, a protein (*e.g.*, gelatin, collagen, *etc.*), a polysaccharide (*e.g.*, starch, amylose, polygalacturonic acid, agar, carrageenan, gum arabic, gellan gum, xanthan gum, pectin, alginic acid, *etc.*), a biodegradable plastic (*e.g.*, polylactic acid, polyhydroxybutyric acid, polyglutamic acid, *etc.*), a hardened fat (*e.g.*, triglyceride or diglyceride of medium chain fatty acids, *etc.* such as butter, margarine, shortening, cacao butter, *etc.*) *etc.*) and a plasticizer (*e.g.*, a sugar, a sugar alcohol, a polyhydric alcohol, *etc.* such as glycerol, sorbitol, polyethylene glycol, *etc.*) as the essential components optionally together with a flavoring agent (*e.g.*, peppermint oil, cinnamon oil, essence or flavor of a fruit such as strawberry, etc.), an antiseptic (*e.g.*, ethyl parahydroxybenzoate, propyl parahydroxybenzoate, *etc.*)*,* a coloring agent (*e.g.,* Yellow No. 4, Yellow No. 5, Red No. 3, Blue No.1, copper chlorofine, *etc.*), an opacifying agent (*e.g.,* titanium dioxide, red iron oxide, *etc.*), a solubility-controlling agent (*e.g*., cellulose acetate phthalate, an alkali metal salt of hydroxypropylmethylcellulose, an alkali metal salt of hydroxymethylcellulose acetate succinate, an alkali alginate, an alkali metal polyacrylate, methylcellulose, carboxymethylcellulose, casein, collagen, agar powder, polyvinyl alcohol, pectin, *etc.*) *etc.*

In the capsule preparation used in the present invention, a capsule shell comprising gelatin as the base is preferred. Particularly, a capsule shell comprising gelatin as the base and contains glycerol as a plasticizer is preferred. It may further contain sorbitol as a plasticizer.

The capsule shell can be produced by using a solution for capsule shell. As the solution for capsule shell, an arbitrary one may be used without specific limitation as long as it contains the components for the capsule shell as described above; can be formed into a thin film in the molten or dissolved state; and can be solidified by cooling and/or drying after the capsule shell formation.

As the additives, any additives commonly used in preparations for oral administration can be used without specific limitation. For example, additives used in soft capsule preparations and oral liquid preparations are preferred. For example, an antiseptic, a preservative, a surfactant, a solubilizer, an emulsifier, a solvent, a pH controller, a buffer, a suspension agent, a thickener, a stabilizer, a dissolution aid, *etc.* can be used. If desired, two or more of these components may be combined and added. Examples of the antiseptic or preservative include benzoic acid, sodium benzoate, sodium sorbate, parabens (*e.g.*, ethyl paraoxybenzoate, butyl paraoxybenzoate, propyl paraoxybenzoate, *etc.*), *etc.* Examples of the surfactant, solubilizer, emulsifier and solvent include hydroxypropylmethylcellulose, polyvinylpyrrolidone, sucrose fatty acid esters, polyoxyethylene hardened castor oils, polysorbate 80, polyethylene glycol, glycerol, ethanol, propylene glycol, water (*e.g.*, distilled water for injection, *etc.*) *etc.* Examples of the pH controller and buffer include an inorganic acid or a base of an alkali, *etc.* such as hydrochloric acid, sodium hydroxide, potassium hydroxide, sodium hydrogen carbonate, *etc.* and an organic acid such as citric acid, malic acid, tartaric acid or succinic acid and salts of the same, *etc.* pH may be controlled by using a method of controlling pH which is commonly used in the technical field of oral preparations or a method similar to it. Examples of the suspending agent and thickener include gum arabic, crystalline cellulose, bee gum, xanthan gum, gelatin, metholose and an edible salt thereof, carmellose and an edible salt thereof, *etc.* Examples of the stabilizer include an edible salt of edetic acid, sodium chloride, an edible salt of pyrosulfuric acid, *etc.* Examples of the dissolution aid include cyclodextrin, arginine, *etc.* These additives are blended in such amounts which are generally used in preparations for oral administration. In addition to the additives as described above, additives described in, for example, Iyakuhin Tenkabutsu Jiten, edited by Nihon Iyakuhin Tenkazai Kyokai (Japan Association of Medicinal Additives), Yakuji Nippo-sha, 2000, *etc.* can be used.

Examples of liquid preparation for internal use for oral administration include a solution, a suspension, an emulsion, a syrup, an elixir, *etc.* These liquid preparations are prepared by dissolving, suspending or emulsifying the agent in a diluent which is commonly used (*e.g.*, purified water, ethanol, a mixture thereof, *etc.*). These liquid preparations may further contain a humectant, a suspending agent, an emulsifier, a sweetener, a flavor, an aroma, a preservative, a buffer, *etc.*

The dosage form for administering the pharmaceutical composition of the present invention is not particularly limited as long as the dosage form allows oral administration. To obtain desirable effects on the disease as described above, it is preferable to use the above-described capsule preparation.

### EXAMPLES

Although clinical effects achieved by the administration of (2R)-2-propyloctanoic acid or a salt thereof in the Dosage and Administration of the present invention to patients of ALS will be described in detail by referring to Examples and Formulation Examples, the present invention is not limited thereto. Further, various changes may be made without departing from the scope of the present invention.

### Example 1:

As a clinical study, double blind placebo-controlled parallel group study was carried out on patients with amyotrophic lateral sclerosis, under the following conditions.

### [STUDY POPULATION]

523 patients with amyotrophic lateral sclerosis who meets the following inclusion criteria and does not meet the exclusion criteria.

### <Inclusion Criteria>

(1) Adult males and females aged 18 to 75 years;
(2) Diagnosis of clinically definite, clinically probable or clinically possible ALS (according to World Federation of Neurology EL Escorial diagnostic criteria, revised according to the Airlie House Conference 1998);
(3) Less than 36 months from onset of muscular weakness;
(4) An SVC (slow vital capacity) of greater than or equal to 70 % of predicted;
(5) Ability to perform SVC manoeuvres in a reliable and reproducible manner;
(6) Treatment with standard riluzole therapy for 3 months prior to study, with liver function test (LFT) results within two times the upper limit of the normal range;
(7) Ability to swallow without the requirement for nasogastric or Percutaneous Endoscopic Gastrostomy feeding;
(8) Clinical laboratory tests within the reference ranges or clinically acceptable to the investigator;
(9) Physical examination results, including vital signs (*e.g.*, blood pressure, heart rate, *etc*.) and 12-lead ECG within the reference ranges or clinically acceptable to the investigator;
(10) Ability to comply with the dosing regimen and visit schedule;
(11) Agreement for themselves or their partner to use an adequate method of contraception throughout the study and for 2 weeks after post study visit. Adequate methods of contraception for themselves or their partner include condoms, diaphragm with spermicide gel, coil (intra-uterine device), surgical sterilisation, vasectomy and abstinence;
(12) Able and willing to give written informed consent.

### <Exclusion Criteria>

(1) Presence of a tracheostomy or non-invasive ventilation;
(2) Requirement for prescription drugs used for potential neuroprotective benefit [*e.g.*, minocycline, carbapenems (*e.g.*, imipenem, meropenem, *etc.*), *etc.* and other drugs (*e.g.*, drugs which can be metabolized by cytochrome P450 2C9 (*e.g.*, phenytoin, warfarin, losartan, torsemide, tienilic acid, dronabinol, glibenclamide, glimepiride, glipizide, tolbutamide, isotretinoin, carbamazepine, sulphamethoxazole, verapamil, *etc.*), etc.)];
(3) A clinically relevant medical history or presence of respiratory diseases or disorders (such as moderate-to-severe asthma and chronic obstructive pulmonary disease) that, in the opinion of the Investigator, may pose an unwarranted risk to the subject or confound the results of the study;
(4) A clinically relevant medical history or presence of cardiovascular, gastrointestinal, renal, hepatic, endocrine/metabolic, neurologic, lymphatic, haematologic, immunologic, musculoskeletal, connective tissue, genito/urinary, psychiatric diseases or disorders that, in the opinion of the Investigator, may pose an unwarranted risk to the subject or confound the results of the study;
(5) Presence or intention of pregnancy and breast feeding (female subjects only);
(6) Males with the intention of fathering a child during the study period;
(7) A history of drug or alcohol abuse (alcoholic subjects who are recovered for at least 2 years will be allowed to enroll in the study);
(8) Subjects who have used any investigational drug and/or participated in any clinical trial within 3 months of entry to this study;
(9) Subjects who have previously received (2R)-2-propyloctanoic acid via the oral route.

### [NUMBER OF SUBJECTS]

(1) Active group ((2R)-2-propyloctanoic acid administered group) : 263 patients;
(2) Placebo group (Placebo administered group) : 260 patients.

### [DOSAGE AND ADMINISTRATION]

Both Active and Placebo groups are administered once a day via the oral route (at approximately the same time each morning).
(I) Active group : (2R)-2-propyloctanoic acid 1200 mg (four soft capsules comprising 300 mg of (2R)-2-propyloctanoic acid per capsule);
(2) Placebo group : Placebo (four placebo capsules (soft capsule which is apparently-indistinguishable from soft capsules comprising 300 mg of (2R)-2-propyloctanoic acid)).

### [DURATION OF TREATMENT]

Both Active and Placebo groups are administered for 12 months.

### [CONCOMITANT THERAPY]

Riluzole was administered to all subjects in Active and Placebo groups in the manner of standard therapy (riluzole 50 mg tablet: one tablet twice a day (before breakfast and dinner), oral daily dose of 100 mg).

### [EFFICACY MEASUREMENTS]

### (1) respiratory function

Evaluation method: Slow vital capacity (SVC) of the subject was measured at the following evaluation times. SVC was measured three times in the sitting position, allowing sufficient recovery time between each manoeuvre. The highest SVC value was recorded.

Evaluation time: 1, 2, 4, 6, 8, 10 and 12 month(s) from the start of administration.

### (2) functional status

Evaluation method: Functional status of the subject was assessed at the following evaluation times using revised amyotrophic lateral sclerosis functional rating scale (ALSFRS-R) shown in Table 1 to 13 below.

Evaluation time: 1, 2, 4, 6, 8, 10 and 12 month(s) from the start of administration.

**Table 1 a. Speech**

| | |
|---|---|
| 4 | Normal |
| 3 | Slight disturbance of speech |
| 2 | Intelligible with repeating |
| 1 | Speech combined with nonvocal communication |
| 0 | Loss of useful speech |

**Table 2 b. Salivation**

| | |
|---|---|
| 4 | Normal |
| 3 | Slight, but definite excess of saliva in mouth; may have nightime drooling |
| 2 | Moderately excessive saliva; may have minimal drooling |
| 1 | Marked excess of saliva with some drooling |
| 0 | Marked drooling; requires constant tissue or handkerchief |

**Table 3 c. Swallowing**

| | |
|---|---|
| 4 | Normal eating habits |
| 3 | Occasional choking |
| 2 | Needs change of dietary content |
| 1 | Needs supplemental tube feeding |
| 0 | Needs parenteral nutrition exclusively |

**Table 4 d. Handwriting**

| | |
|---|---|
| 4 | Normal |
| 3 | All words are legible while slow or sloppy |
| 2 | Not all words are legible |
| 1 | Able to grip pen but unable to write |
| 0 | Unable to grip pen |

**Table 5 e. Choose presence or absence of gastrostomy (subject without gastrostomy) Handling of utensils**

| | |
|---|---|
| 4 | Normal |
| 3 | Slow and clumsy, but no help needed |
| 2 | Cannot use chopsticks but fork and spoon |
| 1 | Needs someone to cut foods but able to eat with fork and spoon |
| 0 | Total dependence |

**Table 6 e. Choose presence or absence of gastrostomy (subject without gastrostomy) Motion of finger**

| | |
|---|---|
| 4 | Normal |
| 3 | Clusmy but able to perform all manipulations |
| 2 | Needs some help with fastening a button and fasteners |
| 1 | Needs assistance for personal care |
| 0 | Unable to perform any fingertip motion |

**Table 7 f. Dressing and hygiene**

| | |
|---|---|
| 4 | Able to dress without obstacle |
| 3 | Independent and complete self-care with effort of decreased efficiency |
| 2 | Intermittent assistance or substitute methods |
| 1 | Needs assistance |
| 0 | Total dependence |

**Table 8 g. Motion in sick bed**

| | |
|---|---|
| 4 | Able without obstacle |
| 3 | Slow and clumsy, but no help needed |
| 2 | Can turn alone or adjust sheets, but with great difficulty |
| 1 | Can initiate, but not turn or adjust sheets alone |
| 0 | Helpless |

**Table 9 h. Walking**

| | |
|---|---|
| 4 | Normal |
| 3 | Slightly difficult to walking |
| 2 | Walks with assistance |
| 1 | Nonambulatory |
| 0 | No purposeful leg movement |

**Table 10 i. Climbing stairs**

| | |
|---|---|
| 4 | Normal |
| 3 | Slow |
| 2 | Mild unsteadiness or fatigue |
| 1 | Needs assistance |
| 0 | Cannot do |

**Table 11 i. Dyspnea**

| | |
|---|---|
| 4 | None |
| 3 | Occurs when walking |
| 2 | Occurs with one or more of the following: eating, bathing, dressing |
| 1 | Occurs when sitting or lying |
| 0 | Needs mechanical respiratory support |

**Table 12 k. Orthopnea**

| | |
|---|---|
| 4 | None |
| 3 | Some difficulty sleeping at night due to shortness of breath |
| 2 | Needs supportive pillows in order to sleep |
| 1 | Can only sleep sitting up |
| 0 | Unable to sleep |

**Table 13 1. Respiratory failure**

| | |
|---|---|
| 4 | None |
| 3 | Intermittent use of ventilator (BiPAP(registered trademark)) |
| 2 | Continuous use of ventilator (BiPAP(registered trademark)) during the night |
| 1 | Continuous use of ventilator (BiPAP(registered trademark)) during the night and day |
| 0 | Invasive mechanical ventilation by intubation or tracheostomy |

### (3) muscle strength

Evaluation method: Muscle strength of the subject was assessed at the following evaluation times using medical research council scale shown in Table 14 below. Shoulder abduction, elbow flexion, wrist extension, hip flexion, knee extension and ankle dorsiflexion will be tested on both the left and right sides of the body.

Evaluation time: 1, 2, 4, 6, 8, 10 and 12 month(s) from the start of administration.

**Table 14 10-point modified MRC scale**

| | |
|---|---|
| 5 | Normal strength |
| 5- | Barely detectable weakness |
| 4+ | Same as 4, but muscle moves joint against more stronger resistance. |
| 4 | Muscle is weak but moves joint against a combination of gravity and moderate resistance. |
| 4- | Same as 4, but muscle moves joint only against minimal resistance. |
| 3+ | The muscle is capable of transient resistance but collapses abruptly. This degree of weakness is difficult to put into words. Muscle moves joint against gravity and an additional small amount of resistance. Muscle cannot bear against sustained resistance and the mechanical range of joint is not complete. |
| 3 | Muscle cannot move against resistance, but moves joint fully against gravity. With the exception of knee extensors, the joint must be moved through the full mechanical range against gravity. If the subject has contractures that limit movement of the joint, the range of movement to the point of contractures is considered as the mechanical range. In the case of knee extensors "full range" will be considered to be within 10 degrees of full extension since it requires considerable strength to extend the knee fully. |
| 3- | Muscle moves joint against gravity but less than 50% of the mechanical range of joint. |
| 2 | Muscle moves joint when gravity is eliminated. |
| 1 | A flicker of movement is seen or felt in the muscle. |
| 0 | No movement |

### (4) index of serious respiratory failure

Evaluation method: An index of serious respiratory failure (presence of morning headaches, orthopnea, dysnea at rest or following mild exertion, excessive daytime hypersomnolence, SVC less than 80% of predicted, presence of other symptoms of respiratory failure, provision of non-invasive ventilation) and blood gas levels of the subject was assessed at the following evaluation times.

Evaluation time: 1, 2, 4, 6, 8, 10 and 12 month(s) from the start of administration.

### (5) survival

Evaluation method: Death was recorded for each subject, if and when it occurs.

Evaluation time: Throughout the study.

### [SAFETY MEASUREMENTS]

### (1) vital signs

Evaluation method: Blood pressure and pulse of the subject were recorded at the following evaluation times.

Evaluation time: 1, 2, 4, 6, 8, 10 and 12 month(s) from the start of administration.

### (2) 12-lead ECG

Evaluation method: 12 lead ECG was conducted at the following evaluation times. Sinus rhythm, RR interval, PR interval, QRS duration, QT/QTc, and so forth were assessed.

Evaluation time: 1, 2, 4, 6, 8, 10 and 12 month(s) from the start of administration.

### (3) weight

Evaluation method: Weight of the subject was measured at the following evaluation times.

Evaluation time: 1, 2, 4, 6, 8, 10 and 12 month(s) from the start of administration.

### (4) clinical laboratory tests

Evaluation method: Clinical laboratory test (haematology, biochemistry and urinalysis) of the subject was conducted at the following evaluation times.

Evaluation time: 1, 2, 4, 6, 8, 10 and 12 month(s) from the start of administration.

### (a) Haematology

red cell count (RBC), haemoglobin (Hb), haematocrit (Ht), white cell count, differential white cell count, platelet count, mean cell volume (MCV), mean corpuscular haemoglobin (MCH), mean corpuscular haemoglobin concentration (MCHC) and activated partial thromboplastin time (APTT).

### (b) Plasma biochemistry

urea, creatinine, uric acid, total bilirubin, sodium, potassium, calcium, phosphorus, chloride, alkaline phosphatase (ALP), aspartate amino transferase (AST), alanine amino transferase (ALT), lactate dehydrogenase (LDH), gamma glutamyl transpeptidase (GGT), creatine kinase (CK), albumin, total protein, cholesterol, triglycerides, creatinine, glucose, α1-globlin, and albumin/globulin ratio.

Testosterone, follicle stimulating hormone (FSH), luteinising hormone (LH) and inhibin B (males only; 6 and 12 months only).

### (c) Urinalysis

pH, urinary protein, sugar urine, ketones, bilirubin, occult blood, nitrites, urinary leukocytes, urobilinogen and specific gravity.

### (5) clinical adverse events

Evaluation method: Incidence and content (*e.g.*, symptom, causal association, *etc.*) of clinical adverse events were monitored for each subject.

Evaluation time; Throughout the study.

### [ANALYSIS]

The effect of oral administration of (2R)-2-propyloctanoic acid on the ALS patients was evaluated by the results of above EFFICACY MEASUREMENTS and SAFETY MEASUREMENTS.

### [RESULT]

Results are shown below.

### <EFFICACY MEASUREMENTS>

Suppression of respiratory disability and improvement in survival rate are shown by oral administration of (2R)-2-propyloctanoic acid in the patient within about 14 months after the onset of muscular weakness (Active group : 55 patients, Placebo group: 54 patients, Both Active and Placebo groups are treated with standard riluzole therapy). Hereinafter, each result is described in detail.

### (a) Suppression of respiratory disability

Compared to before-administration, the slow vital capacity at 12 months from the start of administratioon (% of the predicted value) is reduced by 38.27 % in Active group while reduced 51.10 % in Placebo group. So, the effect of suppression of respiratory disability by (2R)-2-propyloctanoic acid was observed.

Alternatively, in result of estimation for the duration until the time when the slow vital capacity was 50 % or less of predicted value, the slow vital capacity of about 35 % of patients were lowered to 50 % or less of predicted in 12 months in Active group, though the slow vital capacity of about 50 % of patients were lowered to 50 % or less of predicted in 12 months in Placebo group. So, the effect of slowing respiratory disability by (2R)-2-propyloctanoic acid was observed.

### (b) Improvement in survival rate

Mortality in the dosing period is 17 of 54 patients in Placebo group while 9 of 55 patients in Active group. Therefore, the survival rate is 69 % in Placebo group while 84 % in Active group.

### <SAFETY MEASUREMENTS>

Frequency of adverse event is 89 % in Active group while 93 % in Placebo group. Frequency of serious adverse event is 49 % in Active group while 52 % in Placebo group.

### Example 2:

As a clinical study, double blind placebo-controlled parallel group study was carried out on patients with amyotrophic lateral sclerosis, under the following conditions.

### [STUDY POPULATION]

About 400 patients with amyotrophic lateral sclerosis who meets the following inclusion criteria and does not meet the exclusion criteria.

### <Inclusion Criteria>

(1) Adult males and females aged 18 to 75 years;
(2) Diagnosis of clinically definite, clinically probable or clinically possible ALS (according to World Federation of Neurology EL Escorial diagnostic criteria, revised according to the Airlie House Conference 1998);
(3) Less than 14 months from onset of muscular weakness;
(4) An SVC (slow vital capacity) of greater than or equal to 70 % of predicted;
(5) Treatment with standard riluzole therapy for 2 weeks prior to study, with liver function test (LFT) results within two times the upper limit of the normal range;
(6) Ability to swallow without the requirement for nasogastric or Percutaneous Endoscopic Gastrostomy feeding (the score is 3 or more on item "Swallowing" (written in Table 3) of revised amyotrophic lateral sclerosis functional rating scale (ALSFRS-R));
(7) Agreement for themselves or their partner to use an adequate method of contraception throughout the study and for 2 weeks after post study visit. Adequate methods of contraception for themselves or their partner include condoms, diaphragm with spermicide gel, coil (intra-uterine device), surgical sterilisation, vasectomy and abstinence;
(8) Able and willing to give written informed consent.

### <Exclusion Criteria>

(1) Presence of a tracheostomy or non-invasive ventilation (the score is 3 or less on item "Respiratory insufficiency" (written in Table 13) of revised amyotrophic lateral sclerosis functional rating scale (ALSFRS-R));
(2) Requirement for prescription drugs used for potential neuroprotective benefit
   [*e.g.,* minocycline, carbapenems (*e.g.*, imipenem, meropenem, *etc.*), *etc.* and other drugs (*e.g.*, drugs which can be metabolized by cytochrome P450 2C9 (*e.g.,* phenytoin, warfarin, losartan, torsemide, tienilic acid, dronabinol, glibenclamide, glimepiride, glipizide, tolbutamide, isotretinoin, carbamazepine, sulphamethoxazole, verapamil, *etc.*), *etc.*)];
(3) A clinically relevant medical history or presence of respiratory diseases or disorders (such as moderate-to-severe asthma and chronic obstructive pulmonary disease) that, in the opinion of the Investigator, may pose an unwarranted risk to the subject or confound the results of the study;
(4) Presence of intercurrent malignant tumor or presence of previous history thereof within three years;
(5) Presence of another fatal serious disease or presence of previous history thereof within three years;
(6) Presence or intention of pregnancy and breast feeding (female subjects only);
(7) Males with the intention of fathering a child during the study period;
(8) A history of drug or alcohol abuse (alcoholic subjects who are recovered for at least 2 years will be allowed to enroll in the study);
(9) Subjects who have used any investigational drug and /or participated in any clinical trial within 3 months of entry to this study;
(10) Subjects who have previously received (2R)-2-propyloctanoic acid via the oral route.

### [NUMBER OF SUBJECTS]

(1) Active group ((2R)-2-propyloctanoic acid administered group) : about 200 patients;
(2) Placebo group (Placebo administered group): about 200 patients.

### [DOSAGE AND ADMINISTRATION]

Both Active and Placebo group are administered once a day via the oral route (at approximately the same time each morning).
(1) Active group : (2R)-2-propyloctanoic acid 1200 mg (four soft capsules comprising 300 mg of (2R)-2-propyloctanoic acid per capsule);
(2) Placebo group : Placebo (four placebo capsules (soft capsule which is apparently-indistinguishable from soft capsules comprising 300 mg of (2R)-2-propyloctanoic acid)).

### [DURATION OF TREATMENT]

Both Active and Placebo group are administered for 18 months.

### [CONCOMITANT THERAPY]

Riluzole was administered to all subjects in Active and Placebo group in the manner of standard therapy (riluzole 50 mg tablet: one tablet twice a day (before breakfast and dinner), oral daily dose of 100 mg).

### [EFFICACY MEASUREMENTS]

### (1) respiratory function

Evaluation method: Slow vital capacity (SVC) of the subject was measured at the following evaluation times. SVC was measured three times in the sitting position, allowing sufficient recovery time between each manoeuvre. The highest SVC value was recorded.

Evaluation time: 1, 2, 4, 6, 8, 10, 12, 14, 16 and 18 month(s) from the start of administration.

### (2) functional status

Evaluation method: Functional status of the subject was assessed at the following evaluation times using revised amyotrophic lateral sclerosis functional rating scale (ALSFRS-R) shown in above-mentioned Table 1 to 13.

Evaluation time: 1, 2, 4, 6, 8, 10, 12, 14, 16 and 18 month(s) from the start of administration.

### (3) muscle strength

Evaluation method: Muscle strength of the subject was assessed at the following evaluation times using medical research council scale shown in beforementioned Table 14. Shoulder abduction, elbow flexion, wrist extension, hip flexion, knee extension and ankle dorsiflexion will be tested on both the left and right sides of the body.

Evaluation time: 1, 2, 4, 6, 8, 10, 12, 14, 16 and 18 month(s) from the start of administration.

### (4) survival

Evaluation method: Death, bronchotomy, and using noninvasive ventilator were recorded for each subject, if and when it occurs.

Evaluation time: Throughout the study.

### (5) QOL

Evaluation method: QOL of the subject was assessed at the following evaluation times using EQ-5D (Health Policy, 37, 53-72, 1996; available from the internet <URL:www.euroqol.org/>).

Evaluation time: 1, 2, 4, 6, 8, 10, 12, 14, 16 and 18 month(s) from the start of administration.

### [SAFETY MEASUREMENTS]

### (1) vital signs

Evaluation method: Blood pressure and pulse of the subject were recorded at the following evaluation times.

Evaluation time: 1, 2, 4, 6, 8, 10, 12, 14, 16 and 18 month(s) from the start of administration.

### (2) 12-lead ECG

Evaluation method: 12 lead ECG was conducted at the following evaluation times. Sinus rhythm, RR interval, PR interval, QRS duration, QT/QTc, and so forth were assessed.

Evaluation time: 1, 2, 4, 6, 8, 10, 12, 14, 16 and 18 month(s) from the start of administration.

### (3) weight

Evaluation method: Weight of the subject was measured at the following evaluation times.

Evaluation time: 1, 2, 4, 6, 8, 10, 12, 14, 16 and 18 month(s) from the start of administration.

### (4) clinical laboratory tests

Evaluation method: Clinical laboratory test (haematology, biochemistry and urinalysis) of the subject was conducted at the following evaluation times.

### (a) Haematology

red cell count (RBC), haemoglobin (Hb), haematocrit (Ht), white cell count, differential white cell count, platelet count, mean cell volume (MCV), mean corpuscular haemoglobin (MCH), mean corpuscular haemoglobin concentration (MCHC) and activated partial thromboplastin time (APTT).

### (b) Plasma biochemistry

urea, creatinine, uric acid, total bilirubin, sodium, potassium, calcium, phosphorus, chloride, alkaline phosphatase (ALP), aspartate amino transferase (AST), alanine amino transferase (ALT), lactate dehydrogenase (LDH), gamma glutamyl transpeptidase (GGT), creatine kinase (CK), albumin, total protein, cholesterol, triglycerides, creatinine, glucose, α1-globlin, and albumin/globulin ratio.

Testosterone, follicle stimulating hormone (FSH), luteinising hormone (LH) and inhibin B (males only; 6 and 12 months only).

### (c) Urinalysis

pH, urinary protein, sugar urine, ketones, bilirubin, occult blood, nitrites, urinary leukocytes, urobilinogen and specific gravity.

### (5) clinical adverse events

Evaluation method: Incidence and content (*e.g.*, symptom, causal association, *etc.*) of clinical adverse events were monitored for each subject.

Evaluation time: Throughout the study.

### [ANALYSIS]

The effect of oral administration of (2R)-2-propyloctanoic acid on the ALS patients was evaluated by the results of above EFFICACY MEASUREMENTS and SAFETY MEASUREMENTS.

### [RESULT]

Results are shown below.

### <EFFICACY MEASUREMENTS>

(2R)-2-propyloctanoic acid shows its effectiveness on at least one of suppressing muscular weakness, suppressing respiratory disability, extending survival period and improvement in quality-of-life measures, in ALS patient.

### <SAFETY MEASUREMENTS>

(2R)-2-propyloctanoic acid is safe insofar as it is administrated to the ALS patient.

### [Formulation Examples]

Formulation Example I: Production of soft capsules comprising 300 mg of (2R)-2-propyloctanoic acid

Bovine gelatin (20 kg) and conc. glycerol (6 kg) were blended together in the presence of purified water (20 kg) under 70 °C to give a homogeneous solution. The solution and (2R)-2-propyloctanoic acid (0.9 kg) were supplied into a soft capsule encapsulating machine (a rotary soft capsule molding machine Model H-1; KAMATA) to give coarse soft capsules having (2R)-2-propyloctanoic acid encapsulated therein. The coarse soft capsules subjected to tumbler drying (24 °C, 3 hours) and a tray drying (29 °C, 15 to 45 hours) successively. Thus, soft capsules (2100 capsules) comprising 300 mg of (2R)-2-propyloctanoic acid per capsule were obtained.

### INDUSTRIAL APPLICABILITY

Since the agent of the present invention, namely an agent for protecting a motor nerve of a patient with ALS which comprises a combination of (a) (2R)-2-propyloctanoic acid or a salt thereof and (b) a therapeutic agent for ALS, is safe and can control respiratory disability accompanying the progression of ALS, it enables the extension of period until attaching a respirator. Moreover, survival period can be extended and QOL can be improved.

## Claims

1. An agent for protecting a motor nerve of a patient with amyotrophic lateral sclerosis, which comprises a combination of (a) (2R)-2-propyloctanoic acid or a salt thereof and (b) a therapeutic agent for amyotrophic lateral sclerosis.

2. The agent according to claim 1, which is used for suppressing muscular weakness.

3. The agent according to claim 2, which is used for suppressing respiratory disability.

4. The agent according to claim 2, which is used for extending survival period.

5. The agent according to claim 1, which is for oral administration.

6. The agent according to claim 1, wherein the therapeutic agent for amyotrophic lateral sclerosis is riluzole.

7. The agent according to claim 6, wherein the amount per dose of riluzole is 50 mg.

8. The agent according to claim 1, wherein the amount per dose of (2R)-2-propyloctanoic acid or a salt thereof is from 300 mg to 2400 mg in terms of (2R)-2-propyloctanoic acid.

9. The agent according to claim 8, wherein the amount per dose of (2R)-2-propyloctanoic acid or a salt thereof is 300 mg, 600 mg, 900 mg, 1200 mg, 1500 mg, 1800 mg, 2100 mg or 2400 mg in terms of (2R)-2-propyloctanoic acid.

10. The agent according to claim 9, wherein the amount per dose of (2R)-2-propyloctanoic acid or a salt thereof is 1200 mg in terms of (2R)-2-propyloctanoic acid.

11. The agent according to claim 6, wherein the amount per daily dose of (2R)-2-propyloctanoic acid or a salt thereof is from 1200 mg to 2400 mg in terms of (2R)-2-propyloctanoic acid and the amount per daily dose of riluzole is from 50 mg to 200 mg.

12. The agent according to claim 11, wherein the dosing time per day of (2R)-2-propyloctanoic acid or a salt thereof is from once to twice and the dosing time per day of riluzole is from one to four times.

13. An agent for protecting a motor nerve of a patient with amyotrophic lateral sclerosis, which comprises a combination of (a) (2R)-2-propyloctanoic acid or a salt thereof and (b) riluzole and is administered for from one to five years.

14. The agent according to claim 13, which is administered for 12 months to 18 months.

15. The agent according to claim 1 or 13, wherein the patient with amyotrophic lateral sclerosis is a patient within 14 months after the onset of muscular weakness.

16. An agent for suppressing respiratory disability and/or extending survival period of a patient with amyotrophic lateral sclerosis within 14 months after the onset of muscular weakness, which comprises a combination of (a) (2R)-2-propyloctanoic acid which is orally administered once a day in an amount per dose of 1200 mg and (b) riluzole which is orally administered twice a day in an amount per dose of 50 mg during the dosing period of from 12 months to 18 months.

17. An agent which comprises a combination of (a) (2R)-2-propyloctanoic acid which is orally administered once a day in an amount per dose of 1200 mg and (b) riluzole which is orally administered twice a day in an amount per dose of 50 mg during the dosing period of from 12 months to 18 months to suppress respiratory disability and/or extend survival period of a patient with amyotrophic lateral sclerosis within 14 months after the onset of muscular weakness.

18. A method for protecting a motor nerve of a patient with amyotrophic lateral sclerosis, which comprises orally administering a combination of (a) an effective amount of (2R)-2-propyloctanoic acid or a salt thereof and (b) an effective amount of a therapeutic agent for amyotrophic lateral sclerosis to the patient with amyotrophic lateral sclerosis.

19. Use of a combination of (a) (2R)-2-propyloctanoic acid or a salt thereof and (b) a therapeutic agent for amyotrophic lateral sclerosis for producing an agent for protecting a motor nerve which is orally administered to a patient with amyotrophic lateral sclerosis.
